# EUROPÄISCHE PATENTANMELDUNG

(11) **EP 3 639 747 A2**
(43) Veröffentlichungstag der Anmeldung: **22.04.2020**
(21) Anmeldenummer: 19195900.6
(22) Anmeldetag: 06.09.2019
(51) Int. Cl.: A61B 6/03, A61B 5/113, A61B 6/00

(54) **DURCHFÜHREN EINER BILDGEBENDEN MESSUNG EINES PATIENTEN IN EINEM COMPUTERTOMOGRAPHIESYSTEM**

(30) Priorität: 18.10.2018 DE 102018217888
(71) Anmelder: Siemens Healthcare GmbH, 91052 Erlangen (DE)
(72) Erfinder: Hofmann, Christian, 91052 Erlangen (DE)

(57) **Zusammenfassung**

Die Erfindung betrifft ein Verfahren für ein Durchführen einer bildgebenden Messung eines Patienten in einem Computertomographiesystem mit folgenden Schritten:
- Erfassen eines Atemzyklus des Patienten mittels einer Atemerfassungseinheit,
- Bereitstellen des Atemzyklus des Patienten in einer Planungseinheit als Referenzatemzyklus,
- Auswählen zumindest einer Atemphase des Referenzatemzyklus mittels der Planungseinheit, wobei die zumindest eine ausgewählte Atemphase (AP, AP1, AP2) kürzer ist als eine Zyklusdauer des Referenzatemzyklus (R),
- Durchführen der bildgebenden Messung in dem Computertomographiesystem,
-- wobei eine Atmung des Patienten mittels der Atemerfassungseinheit erfasst und als Atemsignalverlauf in eine Steuereinheit übertragen wird,
-- wobei der Atemsignalverlauf mit der zumindest einen ausgewählten Atemphase des Referenzatemzyklus in der Steuereinheit verglichen wird, wodurch ein binäres Vergleichsergebnis generiert wird,
-- wobei in Abhängigkeit von dem binären Vergleichsergebnis eine Datenerfassung in dem Computertomographiesystem ausgelöst wird, wodurch Projektionsdaten in der zumindest einen Atemphase erfasst werden.

## Beschreibung

Die Erfindung betrifft ein Verfahren für ein Durchführen einer bildgebenden Messung eines Patienten in einem Computertomographiesystem, das Computertomographiesystem und ein zugehöriges Computerprogrammprodukt.

Eine Radiotherapieplanung eines Patienten erfordert üblicherweise ein Bereitstellen eines medizinischen Bildes in einer spezifischen Atemphase des Patienten, beispielsweise wenn die Radiotherapieplanung ein aufgrund der Atmung des Patienten bewegtes Zielvolumen, insbesondere ein Lungen- und/oder ein Abdomen-Karzinom, betrifft. In diesem Fall hängt eine Abbildung des Zielvolumens der Radiotherapieplanung in dem medizinischen Bild von der Atmung, insbesondere von der Atemphase, ab. Die Radiotherapieplanung ist vorzugsweise auf die Atemphase des medizinischen Bildes abgestimmt.

Bei einer herkömmlichen bildgebenden Messung werden typischerweise Projektionsdaten für einen gesamten Atemzyklus der Atmung, sprich für jede Atemphase des Atemzyklus, erfasst und lediglich bei einer Rekonstruktion der Projektionsdaten diejenigen Atemphasen herausgefiltert, für welche das medizinische Bild bereitgestellt werden soll.

Der Erfindung liegt die Aufgabe zu Grunde, ein Verfahren für ein Durchführen einer bildgebenden Messung eines Patienten in einem Computertomographiesystem, ein zugehöriges Computertomographiesystem und ein zugehöriges Computerprogrammprodukt anzugeben, bei welchen eine Dosisbelastung des Patienten reduziert wird.

Die Aufgabe wird durch die Merkmale der unabhängigen Ansprüche gelöst. Vorteilhafte Ausgestaltungen sind in den Unteransprüchen beschrieben.

Das erfindungsgemäße Verfahren für ein Durchführen einer bildgebenden Messung eines Patienten in einem Computertomographiesystem weist folgende Schritte auf:
- Erfassen eines Atemzyklus des Patienten mittels einer Atemerfassungseinheit des Computertomographiesystems,
- Bereitstellen des Atemzyklus des Patienten in einer Planungseinheit des Computertomographiesystems als Referenzatemzyklus,
- Auswählen zumindest einer Atemphase des Referenzatemzyklus mittels der Planungseinheit, wobei die zumindest eine ausgewählte Atemphase kürzer ist als eine Zyklusdauer des Referenzatemzyklus,
- Übertragen der zumindest einen ausgewählten Atemphase gemeinsam mit dem Referenzatemzyklus in eine Steuereinheit des Computertomographiesystems,
- Durchführen der bildgebenden Messung in dem Computertomographiesystem,
   -- wobei eine Atmung des Patienten mittels der Atemerfassungseinheit erfasst und als Atemsignalverlauf in die Steuereinheit übertragen wird,
   -- wobei der Atemsignalverlauf mit der zumindest einen ausgewählten Atemphase des Referenzatemzyklus in der Steuereinheit verglichen wird, wodurch ein binäres Vergleichsergebnis generiert wird,
   -- wobei in Abhängigkeit von dem binären Vergleichsergebnis eine Datenerfassung in dem Computertomographiesystem ausgelöst wird, wodurch Projektionsdaten in der zumindest einen Atemphase erfasst werden.

Das Verfahren bietet insbesondere folgende Vorteile:
Vorzugsweise ist eine Messdauer der bildgebenden Messung dadurch verkürzt, dass die Durchführung der bildgebenden Messung beendet werden kann, nachdem die Projektionsdaten in der zumindest einen Atemphase erfasst werden. Die Verkürzung der Messdauer der bildgebenden Messung ist insbesondere vorteilhaft, weil üblicherweise die Messdauer mit Kosten für die bildgebende Messung korreliert. Die verkürzte Messdauer kann insbesondere einen Komfort für den Patienten erhöhen.

Ein weiterer Vorteil kann sein, dass das binäre Vergleichsergebnis vorzugsweise eine Dosismodulation der bildgebenden Messung in Abhängigkeit der Atmung des Patienten ermöglicht. Die Dosismodulation kann ein selektives Erfassen der Projektionsdaten in der zumindest einen Atemphase ermöglichen, was im Vergleich zu einem herkömmlichen kontinuierlichen Erfassen von Projektionsdaten vorteilhaft sein. Das selektive Erfassen kann flexibles Pulsen genannt werden. Die Dosismodulation ermöglicht weiterhin vorzugsweise, dass für bestimmte, insbesondere nicht zu erfassende, Atemphasen des Referenzatemzyklus Projektionsdaten mit einer vergleichsweise niedrigeren Dosisbelastung oder keine Projektionsdaten erfasst werden. Das binäre Vergleichsergebnis ermöglicht insbesondere ein "Gaten" von der zumindest einen Atemphase.

Vorteilhafterweise kann vor dem Durchführen der bildgebenden Messung die zumindest eine Atemphase derart ausgewählt werden, dass für weitere, insbesondere nicht ausgewählte, Atemphasen herkömmliche Projektionsdaten nicht erfasst werden, sondern vorzugsweise lediglich die Projektionsdaten in der zumindest einen Atemphase. Das Auswählen der zumindest einen Atemphase kann vorzugsweise in Abhängigkeit von einer Radiotherapieplanung erfolgen.

Der Vergleich des Atemsignalverlaufs mit der zumindest einen ausgewählten Atemphase des Referenzatemzyklus ermöglicht insbesondere dadurch ein Erhöhen einer Datenqualität der Projektionsdaten, weil vorzugsweise eine Atemabweichung, beispielsweise ein Atemartefakt und/oder eine irreguläre Atmung, ermittelt werden kann, währenddessen vorteilhafterweise keine Projektionsdaten erfasst werden.

Das Verkürzen der Messdauer, das Auswählen der zumindest einen Atemphase und/oder das binäre Vergleichsergebnis ermöglichen insbesondere eine vorteilhaftes Anpassen der Dosisbelastung an den Patienten, besonders vorteilhafterweise eine Reduzierung der Dosisbelastung des Patienten.

Das Durchführen der bildgebenden Messung erfolgt insbesondere in Abhängigkeit von einer klinischen Fragestellung. Die klinische Fragestellung kann die Radiotherapieplanung und/oder die Atmung des Patienten betreffen. Beispielsweise können die Projektionsdaten in der zumindest einen Atemphase für ein Bereitstellen von einer Ventilationskarte und/oder von einem atemkorrelierten volumetrischen Bilddatensatz des Patienten verwendet werden.

Die Atemerfassungseinheit kann einen Atemgurt, ein Spirometer und/oder eine Kamera umfassen. Die Kamera kann insbesondere eine 3D-Kamera und/oder eine Video-Kamera und/oder Infrarot-Kamera sein. Grundsätzlich ist es denkbar, dass für das Erfassen des Atemzyklus und für das Erfassen der Atmung verschiedene Einheiten der Atemerfassungseinheit verwendet werden. Der Patient kann für das Erfassen des Atemzyklus auf einer Patientenliege des Computertomographiesystems gelagert werden und dort für das Durchführen der bildgebenden Messung verbleiben. In anderen Worten wird der Patient vorzugsweise zwischen dem Erfassen des Atemzyklus und dem Durchführen der bildgebenden Messung nicht umgelagert, sondern verbleibt insbesondere auf der Patientenliege.

Typischerweise wird eine Atmung des Patienten mittels der Atemerfassungseinheit mindestens über eine Zyklusdauer der Atmung des Patienten erfasst. Die Zyklusdauer beträgt typischerweise 2π, wenn die Atmung phasenwinkelabhängig oder zyklisch parametrisiert wird. Der Atemzyklus umfasst insbesondere jene Atemphasen, welche gemeinsam die Zyklusdauer bilden. Grundsätzlich ist es denkbar, dass der Atemzyklus solange erfasst wird, bis eine bestimmte Atemphase des Atemzyklus wiederholt erfasst wird. Die bestimmte Atemphase des Atemzyklus kann Einatmen und/oder Ausatmen beschreiben. Vorzugsweise wird der Atemzyklus des Patienten solange erfasst, bis der Referenzatemzyklus vorzugsweise die Zyklusdauer der Atmung aufweist. Der Atemzyklus wird vorzugsweise während einer insbesondere regulären Atmung des Patienten, typischerweise ohne das Atemartefakt und/oder die Atemabweichung, erfasst. Das Bereitstellen des Atemzyklus des Patienten kann ein Übertragen des erfassten Atemzyklus in die Planungseinheit umfassen. Der Referenzatemzyklus wird typischerweise auf einer Anzeigeeinheit der Planungseinheit angezeigt. Das Bereitstellen des Atemzyklus umfasst insbesondere das Anzeigen. Der Referenzatemzyklus umfasst beispielsweise die Zyklusdauer. Typischerweise entspricht eine Zyklusdauer des Referenzatemzyklus der Zyklusdauer der Atmung des Patienten. Beispielsweise werden bestimmte wiederkehrende Atemphasen des Atemzyklus lediglich einmal in der Planungseinheit bereitgestellt. Der Referenzatemzyklus umfasst typischerweise 2π, wenn der Referenzatemzyklus phasenwinkelabhängig oder zyklisch parametrisiert wird. Der Referenzatemzyklus weist insbesondere die Zyklusdauer auf. Grundsätzlich ist es denkbar, dass der Referenzatemzyklus kürzer oder länger als die Zyklusdauer bereitgestellt wird. Das Bereitstellen kann ein Kürzen und/oder ein Interpolieren und/oder ein Extrapolieren und/oder ein Modellieren und/oder ein Filtern des erfassten Atemzyklus umfassen. Der Referenzatemzyklus ist vorzugsweise atemartefaktfrei.

Die zumindest eine ausgewählte Atemphase ist kürzer als eine Zyklusdauer des Referenzatemzyklus. Insbesondere umfasst die zumindest eine ausgewählte Atemphase weniger Phasenwinkel als die Zyklusdauer des Referenzatemzyklus, insbesondere weniger als 2π. Dies ist insofern vorteilhaft, weil die Projektionsdaten erfasst werden können bevor die Zyklusdauer des Referenzatemzyklus endet, wodurch das Durchführen der bildgebenden Messung vorzugsweise früher beendet werden kann.

Das Auswählen der zumindest einen Atemphase erfolgt vorzugsweise in Abhängigkeit von der klinischen Fragestellung, beispielsweise von der Radiotherapieplanung. Das Auswählen erfolgt beispielsweise automatisch oder durch einen Nutzer der Planungseinheit, wofür die Planungseinheit beispielsweise Eingabemittel aufweisen kann. Das Auswählen der zumindest einen Atemphase kann beispielsweise derart erfolgen, dass der Nutzer mittels der Eingabemittel die zumindest eine Atemphase auf der Anzeigeeinheit relativ zum Referenzatemzyklus markiert, insbesondere per drag'n'drop. Es ist denkbar, dass der Referenzatemzyklus in verschiedene Atemphasen segmentiert wird, bevor der Nutzer die zumindest eine Atemphase markiert. Alternativ oder zusätzlich kann der Nutzer beispielsweise die zumindest eine Atemphase aus einer Liste mit verschiedenen Atemphasen gemäß der Segmentierung des Referenzatemzyklus auswählen. Die zumindest eine ausgewählte Atemphase kann beispielsweise relativ zum Referenzatemzyklus ausgewählt sein. Die zumindest eine ausgewählte Atemphase deckt typischerweise den Referenzatemzyklus, insbesondere die Zyklusdauer des Referenzatemzyklus, zumindest teilweise ab.

Die zumindest eine ausgewählte Atemphase wird vorzugsweise gemeinsam mit dem Referenzatemzyklus von der Planungseinheit in die Steuereinheit übertragen. Das Übertragen kann ein Abrufen der zumindest einen ausgewählten Atemphase gemeinsam mit dem Referenzatemzyklus von der Planungseinheit umfassen. Das Computertomographiesystem kann eine Speichereinheit aufweisen, in welcher beispielsweise der Referenzatemzyklus, der erfasste Atemzyklus und/oder die zumindest eine ausgewählte Atemphase zwischengespeichert werden kann. Die Speichereinheit ist insbesondere mit der Atemerfassungseinheit, der Planungseinheit und/oder der Steuereinheit des Computertomographiesystems, beispielsweise über ein Netzwerk, verbunden.

Die Steuereinheit ist insbesondere für ein Ausführen eines Messprotokolls ausgebildet, welches das Durchführen der bildgebenden Messung des Patienten in dem Computertomographiesystem in Abhängigkeit von der zumindest einen ausgewählten Atemphase und von dem Referenzzyklus abbildet. Grundsätzlich ist es denkbar, dass mittels der Planungseinheit das Messprotokoll festgelegt wird, welches insbesondere die bildgebende Messung parametrisiert. Das Messprotokoll kann beispielsweise einen Messbereich mit einer oder mehreren z-Positionen, eine Kontrastmittelgabe, eine Matrixgröße, einen Tischvorschub, einen Röhrenstrom, eine Röhrenspannung, einen Rekonstruktionskern und/oder eine Röntgenstrahlkollimation insbesondere als Parametrisierung aufweisen. Die zumindest eine ausgewählte Atemphase und/oder der Referenzatemzyklus können ein Teil des Messprotokolls sein. Das Messprotokoll wird beispielsweise durch den Nutzer oder automatisch in Abhängigkeit von der klinischen Fragestellung festgelegt. In Abhängigkeit der Parametrisierung des Messprotokolls variiert typischerweise die Messdauer der bildgebenden Messung. Das Messprotokoll kann insbesondere in Messprotokollprogrammcodemitteln abgebildet sein, welche vorzugsweise in der Steuereinheit ausgeführt werden können.

Die Steuereinheit kann Steuerinformationen umfassen, welche beispielsweise in einem Prozessor und/oder in einem Arbeitsspeicher einer Recheneinheit zumindest teilweise ausgeführt werden können. Die Steuerinformationen können die Messprotokollprogrammcodemittel umfassen.

Im Allgemeinen weist ein Atemsignal typischerweise eine Amplitude und einen Zeitpunkt auf. Die Atemerfassungseinheit wird typischerweise für das Erfassen des Atemzyklus des Patienten und für das Erfassen der Atmung des Patienten verwendet. Das Erfassen der Atmung des Patienten umfasst insbesondere ein regelmäßiges und/oder ein kontinuierliches Erfassen von Atemsignalen der Atmung. Das Erfassen der Atmung kann von einer Abtastrate abhängen. Die Abtastrate beträgt insbesondere zwischen 1 und 100 Hz, besonders vorteilhafterweise zwischen 40 und 60 Hz. Typischerweise werden je Zyklusdauer der Atmung mehrere Atemsignale erfasst. Der Atemsignalverlauf umfasst typischerweise die mehreren Atemsignale, beispielsweise in einem Vektor oder in einer Liste. Der Atemsignalverlauf umfasst insbesondere Atemsignale mehrerer Atemzyklen der Atmung. Der Atemsignalverlauf wird typischerweise laufend während dem Durchführen der bildgebenden Messung aktualisiert. Die Atmung des Patienten wird vorzugsweise online oder live während dem Durchführen der bildgebenden Messung erfasst. Der Atemsignalverlauf wird beispielsweise von der Atemerfassungseinheit über das Netzwerk in die Steuereinheit übertragen.

Das Vergleichen des Atemsignalverlaufs mit der zumindest einen ausgewählten Atemphase des Referenzatemzyklus kann ein Kürzen und/oder ein Interpolieren und/oder ein Extrapolieren und/oder ein Modellieren und/oder ein Filtern des Atemsignalverlaufs umfassen. Das Vergleichen umfasst insbesondere ein Festlegen von Kriterien basierend auf der zumindest einen ausgewählten Atemphase. Das Generieren des binären Vergleichsergebnisses umfasst insbesondere ein Überprüfen, ob der Atemsignalverlauf relativ zu der zumindest einen ausgewählten Atemphase die Kriterien erfüllt oder nicht. Die Kriterien umfassen insbesondere einen Toleranzbereich für eine Abweichung zwischen dem Atemsignalverlauf und der zumindest einen ausgewählten Atemphase. Die Abweichung kann insbesondere eine zeitliche Abweichung zwischen dem Atemsignalverlauf, insbesondere einem jüngsten Atemsignal des Atemsignalverlaufs, und der zumindest einen ausgewählten Atemphase umfassen. Beispielsweise kann ein Zeitpunkt des jüngsten Atemsignals des Atemsignalverlaufs relativ zum Atemzyklus des Patienten ermittelt und mit der zumindest einen ausgewählten Atemphase verglichen werden, wobei die zeitliche Abweichung ermittelt und mit dem Toleranzbereich verglichen wird, wodurch das binäre Vergleichsergebnis generiert wird. Alternativ oder zusätzlich kann die Abweichung eine Abweichung in einer Regularität des Atemsignalverlaufs und in einer Regularität der zumindest einen ausgewählten Atemphase umfassen. Die Regularität kann insbesondere mittels dem Modellieren und/oder dem Filtern und/oder dem Interpolieren ermittelt und/oder quantifiziert werden. Das Vergleichen kann ein Vergleichen der quantifizierten Regularität umfassen. Beispielsweise kann der Toleranzbereich derart festgelegt werden, dass aufgrund der irregulären Atmung des Patienten keine Datenerfassung und/oder lediglich während der regulären Atmung des Patienten die Datenerfassung erfolgt. Aufgrund der irregulären Atmung und/oder des Atemartefaktes und/oder der Atemabweichung kann die Regularität niedriger sein als im Vergleich zu der Regularität während der regelmäßigen Atmung. In einer besonders bevorzugten Ausführung kann die Abweichung die zeitliche Abweichung und die Abweichung in der Regularität umfassen.

Das binäre Vergleichsergebnis weist typischerweise ein Wahr für die Datenerfassung auf, wenn der Atemsignalverlauf relativ zu der zumindest einen ausgewählten Atemphase die Kriterien erfüllt. Das binäre Vergleichsergebnis kann unterschiedliche Werte, insbesondere Wahr oder Falsch, aufweisen und/oder insbesondere während dem Durchführen der bildgebenden Messung mehrmals wechseln. Das Wechseln des binären Vergleichsergebnisses während der bildgebenden Messung kann von der Abtastrate beim Erfassen der Atmung des Patienten abhängen und/oder von einer Takteinheit der Steuereinheit getaktet werden. Grundsätzlich ist es denkbar, dass in Abhängigkeit von der Abtastrate oder der Takteinheit das binäre Vergleichsergebnis mehrmals Wahr oder Falsch aufweist bevor es wechselt. Das binäre Vergleichsergebnis kann beispielsweise für jeden Vergleich der Atemsignale des Atemsignalverlaufs ein Wahr oder ein Falsch aufweisen, insbesondere in einem Vektor oder in einer Liste.

Die Datenerfassung erfolgt beispielsweise mittels einer Röntgenröhre und einem Röntgendetektor. Die Röntgenröhre wird vorzugsweise in Abhängigkeit von dem binären Vergleichsergebnis angeschaltet, wobei typischerweise Röntgenstrahlung emittiert wird, oder abgeschaltet. Grundsätzlich ist es denkbar, dass die Röntgenstrahlung mittels einer Blende im Strahlengang des Computertomographiesystems angeschaltet oder abgeschaltet wird. Die Datenerfassung erfolgt typischerweise mittels der Röntgenstrahlung. Das Auslösen der Datenerfassung entspricht typischerweise dem Anschalten der Röntgenstrahlung, insbesondere wenn das binäre Vergleichsergebnis Wahr aufweist. Die Datenerfassung wird typischerweise beendet oder nicht ausgelöst, wobei beispielsweise die Röntgenstrahlung abgeschaltet wird, wenn das binäre Vergleichsergebnis Falsch aufweist. Wenn das binäre Vergleichsergebnis Falsch für die Datenerfassung aufweist, wird üblicherweise die Röntgenstrahlung, beispielsweise mittels der Blende oder der Röntgenröhre, abgeschaltet. In diesem Fall wird die Röntgenstrahlung typischerweise außerhalb des Patienten vorzugsweise vollständig absorbiert. In anderen Worten wird der Patient typischerweise nicht mittels der Röntgenstrahlung durchdrungen, wenn das binäre Vergleichsergebnis ein Falsch aufweist, wodurch vorzugsweise die Dosisbelastung aufgrund der Röntgenstrahlung verringert wird. Die Dosisbelastung des Patienten wird vorzugsweise nur während der zumindest einen ausgewählten Atemphase des Patienten erhöht.

Das Anschalten und/oder das Abschalten der Röntgenstrahlung erfolgt vorzugsweise innerhalb von 1 s, besonders vorteilhafterweise innerhalb von 0,1 s oder in einem bevorzugten Fall instantan nach dem Auslösen der Datenerfassung. Die Steuereinheit löst typischerweise in Abhängigkeit von dem binären Vergleichsergebnis die Datenerfassung aus. Vorteilhafterweise kann die Steuereinheit das binäre Vergleichsergebnis nach dem Abtasten des jüngsten Atemsignals generieren und die Datenerfassung auslösen oder die Röntgenstrahlung abschalten, bevor gemäß der Abtastrate ein weiteres Atemsignal der Atmung erfasst und/oder in die Steuereinheit für das Vergleichen übertragen wird. Das Auslösen der Datenerfassung und/oder das Abschalten der Röntgenstrahlung kann mehrmals erfolgen, wenn das binäre Vergleichsergebnis mehrmals wechselt. Das Anschalten und/oder das Abschalten der Röntgenstrahlung erfolgen typischerweise gemäß der Abtastrate beim Erfassen der Atmung des Patienten und/oder gemäß der Takteinheit der Steuereinheit.

Typischerweise wird die Datenerfassung für einen Datenerfassungszeitraum ausgelöst, welcher beispielsweise in Abhängigkeit von der klinischen Fragestellung festgelegt wird und/oder mittels des weiteren Atemsignals nach dem Atemsignalverlauf verlängert oder beendet wird.

Während der Datenerfassung werden typischerweise die Projektionsdaten erfasst, wofür üblicherweise die Röntgenstrahlung den Patienten zumindest teilweise durchdringen und/oder zumindest teilweise von dem Röntgendetektor erfasst werden. Die Projektionsdaten sind üblicherweise Rohdaten des Computertomographiesystems. Die Projektionsdaten können beispielsweise in der Speichereinheit des Computertomographiesystems zwischengespeichert werden und/oder in ein Radiologieinformationssystem und/oder in ein PACS-Bildarchivierungssystem übertragen werden.

Eine Ausführungsform sieht vor, dass nach dem Durchführen der bildgebenden Messung ein medizinisches Bild in der zumindest einen Atemphase unter Verwendung der Projektionsdaten rekonstruiert und bereitgestellt wird. Das Rekonstruieren der Projektionsdaten kann beispielsweise in der Steuereinheit erfolgen. Das Rekonstruieren kann in Rekonstruktionsprogrammcodemittel abgebildet sein. Für das Rekonstruieren kann die Steuereinheit beispielsweise die Projektionsdaten von dem Röntgendetektor und/oder aus der Speichereinheit und/oder dem Radiologieinformationssystem und/oder dem PACS-Bildarchivierungssystem abrufen. Das Rekonstruieren erfolgt beispielsweise unter Verwendung des Rekonstruktionskerns und/oder einer gefilterten Rückprojektion und/oder einer iterativen Rekonstruktionsvorschrift. Das medizinische Bild wird beispielsweise in dem Radiologieinformationssystem und/oder im PACS-Bildarchivierungssystem und/oder auf der Planungseinheit bereitgestellt. Das Bereitstellen auf der Planungseinheit kann ein Anzeigen des medizinischen Bildes für den Nutzer der Planungseinheit umfassen. Das medizinische Bild weist vorzugsweise eine höhere Bildqualität auf und kann vorteilhafterweise schneller als bei einer herkömmlichen medizinischen Bildgebung bereitgestellt werden. Das medizinische Bild liegt beispielsweise gemäß einem DICOM-Bildformat vor.

Eine Ausführungsform sieht vor, dass die zumindest eine Atemphase eine erste Atemphase und eine zweite Atemphase umfasst, welche unterschiedliche Atemphasen des Referenzatemzyklus abdecken, und wobei die Datenerfassung gemäß der ersten Atemphase und gemäß der zweiten Atemphase ausgelöst wird. In diesem Fall ist typischerweise eine Summe der ersten Atemphase und der zweiten Atemphase kürzer als die Zyklusdauer des Referenzatemzyklus. Die unterschiedlichen Atemphasen können das Einatmen, das Ausatmen und/oder eine beliebige Anzahl an Zwischenphasen zwischen dem Einatmen und dem Ausatmen aufweisen. Das Übertragen der zumindest einen ausgewählten Atemphase und/oder das Durchführen der bildgebenden Messung und/oder das Vergleichen der zumindest einen ausgewählten Atemphase betreffen typischerweise die erste Atemphase und die zweite Atemphase.

Das binäre Vergleichsergebnis kann beispielsweise zwischen der ersten Atemphase und der zweiten Atemphase auf Falsch wechseln, wobei während der ersten Atemphase und/oder der zweiten Atemphase das binäre Vergleichsergebnis Wahr aufweist. Wenn die erste Atemphase und die zweite Atemphase die unterschiedlichen Atemphasen des Referenzatemzyklus abdecken, ist üblicherweise eine Schnittmenge zwischen der ersten Atemphase und der zweiten Atemphase leer.

Eine Ausführungsform sieht vor, dass die bildgebende Messung in einem Messbereich mit einer ersten z-Position und einer zweiten z-Position durchgeführt wird und wobei die Projektionsdaten gemäß der zumindest einen Atemphase an der ersten z-Position und an der zweiten z-Position erfasst werden. Das Übertragen der zumindest einen ausgewählten Atemphase und/oder das Durchführen der bildgebenden Messung und/oder das Vergleichen der zumindest einen ausgewählten Atemphase betreffen typischerweise die erste z-Position und die zweite z-Position. Die erste z-Position und die zweite z-Position werden beispielsweise mittels des Messprotokolls festgelegt. Diese Ausführungsform ist insbesondere vorteilhaft, weil die Projektionsdaten an verschiedenen z-Positionen in der zumindest einen Atemphase erfasst werden können, wodurch ein medizinisches Bild an der ersten z-Position und an der zweiten z-Position, welches einen volumetrischen medizinischen Bilddatensatz aufweisen kann, rekonstruiert und/oder bereitgestellt werden kann.

Eine Ausführungsform sieht vor, dass die zumindest eine Atemphase die erste Atemphase und die zweite Atemphase umfasst, welche die unterschiedliche Atemphasen des Referenzatemzyklus abdecken, wobei die bildgebende Messung in dem Messbereich mit der ersten z-Position und der zweiten z-Position durchgeführt wird, wobei die Datenerfassung gemäß der ersten Atemphase und gemäß der zweiten Atemphase ausgelöst wird und wobei die Projektionsdaten gemäß der zumindest einen Atemphase an der ersten z-Position und an der zweiten z-Position erfasst werden. Diese Ausführungsform ermöglicht insbesondere das Erfassen der Projektionsdaten derart, dass der atemkorrelierte volumetrische Bilddatensatz rekonstruiert und bereitgestellt werden kann. Der atemkorrelierte Bilddatensatz basiert insbesondere auf Projektionsdaten, welche an unterschiedlichen z-Positionen in mehreren Atemphasen des Referenzatemzyklus erfasst werden.

Eine Ausführungsform sieht vor, dass für das Vergleichen der Referenzatemzyklus zyklisch in Abhängigkeit einer Änderungsrate des Referenzatemzyklus und einer Amplitude des Referenzatemzyklus parametrisiert wird und wobei der Atemsignalverlauf zyklisch in Abhängigkeit einer Änderungsrate des Atemsignalverlaufs und einer Amplitude des Atemsignalverlaufs parametrisiert wird. Die Änderungsrate beschreibt insbesondere eine Geschwindigkeit und/oder kann beispielsweise mittels einer Amplitudenänderung pro Zeiteinheit berechnet werden. Die Amplitude des Referenzatemzyklus und/oder die Amplitude des Atemsignalverlaufs kann beispielsweise normiert und/oder gewichtet werden. Das zyklische Parametrisieren ist insbesondere vorteilhaft, weil der Referenzatemzyklus die Zyklusdauer vorzugsweise aufweist und/oder weil die Atmung des Patienten typischerweise periodisch ist. Das zyklische Parametrisieren kann dem phasenwinkelabhängigen Parametrisieren entsprechen.

Eine Ausführungsform sieht vor, dass das Vergleichen des Atemsignalverlaufs mit der zumindest einen ausgewählten Atemphase des Referenzatemzyklus ein Berechnen einer Tangente des Atemsignalverlaufs umfasst. Das Berechnen der Tangente wird insbesondere dadurch ermöglicht, dass der Atemsignalverlauf mehr als ein Atemsignal aufweist. Für das Berechnen der Tangente werden typischerweise mehrere Atemsignale des Atemsignalverlaufs berücksichtigt, welche beispielsweise gemittelt oder gewichtet werden. Typischerweise weist der Atemsignalverlauf lediglich am Beginn der bildgebenden Messung nur ein Atemsignal auf.

Eine Ausführungsform sieht vor, dass das Vergleichen des Atemsignalverlaufs mit der zumindest einen ausgewählten Atemphase des Referenzatemzyklus ein Berechnen eines Vektors zwischen dem Atemsignalverlauf und einem Mittelpunkt des Referenzatemzyklus umfasst. Der Mittelpunkt des Referenzatemzyklus kann von einem Integral unter dem Referenzatemzyklus abhängen. Insbesondere wenn der Referenzatemzyklus zyklisch parametrisiert wird, kann der Mittelwert aus y- und x-Werten des Referenzatemzyklus gebildet werden. Grundsätzlich ist es denkbar, dass die y- und x-Werte des Referenzatemzyklus unterschiedlich gewichtet werden, wodurch der Mittelpunkt des Referenzatemzyklus berechnet wird.

Eine Ausführungsform sieht vor, dass das Vergleichen des Atemsignalverlaufs mit der zumindest einen ausgewählten Atemphase des Referenzatemzyklus ein Festlegen eines Winkelbereichs gemäß der zumindest einen ausgewählten Atemphase des Referenzatemzyklus umfasst und wobei der Winkelbereich in Abhängigkeit der Änderungsrate des Referenzatemzyklus und der Amplitude des Referenzatemzyklus festgelegt wird. Das Festlegen des Winkelbereichs umfasst insbesondere ein Umrechnen der zumindest einen ausgewählten Atemphase in den Winkelbereich, insbesondere wenn der Referenzatemzyklus zyklisch parametrisiert wird.

Eine Ausführungsform sieht vor, dass ein Tangentenwinkelbereich in Abhängigkeit von dem Winkelbereich um eine Referenztangente des Referenzatemzyklus festgelegt wird, wobei beim Vergleichen die Tangente des Atemsignalverlaufs und der Tangentenwinkelbereich eingehen. Diese Ausführungsform ist insbesondere vorteilhaft, weil die Tangente und der Tangentenwinkelbereich typischerweise schnell verglichen werden können.
Eine Ausführungsform sieht vor, dass ein Vektorwinkelbereich in Abhängigkeit von dem Winkelbereich und dem Mittelpunkt des Referenzatemzyklus festgelegt wird und wobei beim Vergleichen der Vektor zwischen dem Atemsignalverlauf und dem Mittelpunkt des Referenzatemzyklus sowie der Vektorwinkelbereich eingehen. Diese Ausführungsform bietet insbesondere ein schnelles Vergleichen als Vorteil.

Eine Ausführungsform sieht vor, dass der Tangentenwinkelbereich und der Vektorwinkelbereich festgelegt werden und dass beim Vergleichen die Tangente des Atemsignalverlaufs und der Tangentenwinkelbereich und der Vektor zwischen dem Atemsignalverlauf und dem Mittelpunkt des Referenzatemzyklus sowie der Vektorwinkelbereich eingehen. Diese Ausführungsform ist insbesondere vorteilhaft, weil je mehr Kriterien überprüft werden, desto besser typischerweise die Datenqualität der Projektionsdaten ist.

Das Computertomographiesystem weist die Atemerfassungseinheit, die Planungseinheit und die Steuereinheit auf. Typischerweise weist das Computertomographiesystem die Röntgenröhre und den Röntgendetektor auf. Das Computertomographiesystem ist vorzugsweise gemäß dem Verfahren für das Durchführen der bildgebenden Messung des Patienten ausgebildet.

Ein Computerprogrammprodukt, welches direkt in einen Speicher der Recheneinheit ladbar ist, weist Programmcodemittel auf, um das Verfahren für das Durchführen der bildgebenden Messung des Patienten in dem Computertomographiesystem auszuführen, wenn das Computerprogrammprodukt in der Recheneinheit ausgeführt wird. Die Recheneinheit kann Teil des Computertomographiesystems sein und/oder den Prozessor und/oder den Arbeitsspeicher aufweisen.

Das Computerprogrammprodukt kann ein Computerprogramm sein oder ein Computerprogramm umfassen. Das Computerprogrammprodukt weist insbesondere die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist vorzugsweise derart konfiguriert, dass die Recheneinheit mittels des Computerprogrammprodukts die erfindungsgemäßen Verfahrensschritte ausführen kann. Die Programmcodemittel können insbesondere in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network- (WAN) bzw. einer (Wireless-)Local-Area-Network-Verbindung (WLAN oder LAN) mit der Recheneinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Bei der Beschreibung der Vorrichtung erwähnte Merkmale, Vorteile oder alternative Ausführungsformen sind ebenso auf das Verfahren zu übertragen und umgekehrt. Mit anderen Worten können Ansprüche auf das Verfahren mit Merkmalen der Vorrichtung weitergebildet sein und umgekehrt. Insbesondere kann die erfindungsgemäße Vorrichtung in dem Verfahren verwendet werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 ein Verfahren für ein Durchführen einer bildgebenden Messung eines Patienten in einem Computertomographiesystem in einem ersten Ausführungsbeispiel,
Fig. 2 das Verfahren in einem zweiten Ausführungsbeispiel,
Fig. 3 eine schematische Abbildung des Referenzatemzyklus in einem dritten Ausführungsbeispiel,
Fig. 4 eine schematische Abbildung des Referenzatemzyklus und des Atemsignalverlaufs in einem vierten Ausführungsbeispiel und
Fig. 5 ein Computertomographiesystem.

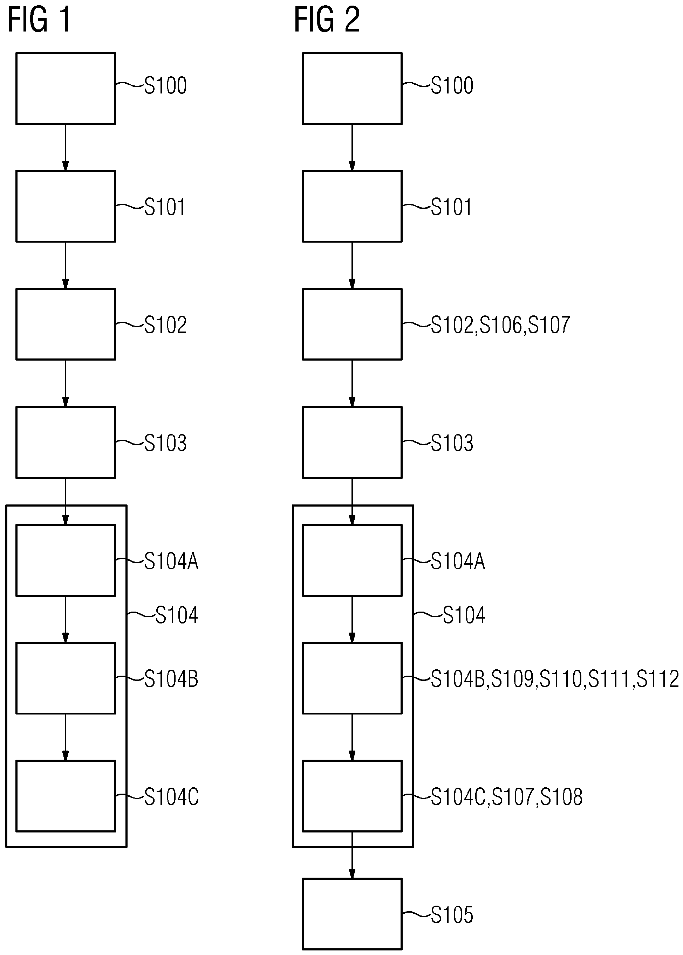

**Fig. 1** zeigt ein Flussdiagramm eines Verfahrens für ein Durchführen einer bildgebenden Messung eines Patienten P in einem Computertomographiesystem 10 in einem ersten Ausführungsbeispiel.

Verfahrensschritt S100 kennzeichnet ein Erfassen eines Atemzyklus des Patienten P mittels einer Atemerfassungseinheit 11 des Computertomographiesystems 10.

Verfahrensschritt S101 kennzeichnet ein Bereitstellen des Atemzyklus des Patienten P in einer Planungseinheit 12 des Computertomographiesystems 10 als Referenzatemzyklus R.

Verfahrensschritt S102 kennzeichnet ein Auswählen zumindest einer Atemphase AP, AP1, AP2 des Referenzatemzyklus R mittels der Planungseinheit 12.

Verfahrensschritt S103 kennzeichnet ein Übertragen der zumindest einen ausgewählten Atemphase AP, AP1, AP2 gemeinsam mit dem Referenzatemzyklus R in eine Steuereinheit 13 des Computertomographiesystems 10.

Verfahrensschritt S104 kennzeichnet ein Durchführen der bildgebenden Messung in dem Computertomographiesystem 10.

Verfahrensschritt S104A kennzeichnet, dass eine Atmung des Patienten P mittels der Atemerfassungseinheit 11 erfasst und als Atemsignalverlauf ASV in die Steuereinheit 13 übertragen wird.

Verfahrensschritt S104B kennzeichnet, dass der Atemsignalverlauf ASV mit der zumindest einen ausgewählten Atemphase AP, AP1, AP2 des Referenzatemzyklus R in der Steuereinheit 13 verglichen wird, wodurch ein binäres Vergleichsergebnis generiert wird.

Verfahrensschritt S104C kennzeichnet, dass in Abhängigkeit von dem binären Vergleichsergebnis eine Datenerfassung in dem Computertomographiesystem 10 ausgelöst wird, wodurch Projektionsdaten in der zumindest einen Atemphase AP, AP1, AP2 erfasst werden.

**Fig. 2** zeigt ein Flussdiagramm des Verfahrens für das Durchführen der bildgebenden Messung des Patienten P in dem Computertomographiesystem 10 in einem zweiten Ausführungsbeispiel.

Verfahrensschritt S105 kennzeichnet, dass nach dem Durchführen der bildgebenden Messung ein medizinisches Bild in der zumindest einen Atemphase AP, AP1, AP2 unter Verwendung der Projektionsdaten rekonstruiert und bereitgestellt wird.

Verfahrensschritt S106 kennzeichnet, dass die zumindest eine ausgewählte Atemphase AP, AP1, AP2 kürzer ist als eine Zyklusdauer des Referenzatemzyklus R.

Verfahrensschritt S107 kennzeichnet, dass die zumindest eine Atemphase AP, AP1, AP2 eine erste Atemphase AP1 und eine zweite Atemphase AP2 umfasst, welche unterschiedliche Atemphasen des Referenzatemzyklus R abdecken, und dass die Datenerfassung gemäß der ersten Atemphase AP1 und gemäß der zweiten Atemphase AP2 ausgelöst wird.

Verfahrensschritt S108 kennzeichnet, dass die bildgebende Messung in einem Messbereich mit einer ersten z-Position und einer zweiten z-Position durchgeführt wird und dass die Projektionsdaten gemäß der zumindest einen Atemphase AP, AP1, AP2 an der ersten z-Position und an der zweiten z-Position erfasst werden.

Das zweite Ausführungsbeispiel, insbesondere Verfahrensschritt S107 und Verfahrensschritt S108, zeigt vorteilhafterweise das Erfassen der Projektionsdaten derart, dass der atemkorrelierte volumetrische Bilddatensatz rekonstruiert und bereitgestellt werden kann.

Verfahrensschritt S109 kennzeichnet, dass für das Vergleichen der Referenzatemzyklus R zyklisch in Abhängigkeit einer Änderungsrate des Referenzatemzyklus R und einer Amplitude des Referenzatemzyklus R parametrisiert wird und dass der Atemsignalverlauf ASV zyklisch in Abhängigkeit einer Änderungsrate des Atemsignalverlaufs ASV und einer Amplitude des Atemsignalverlaufs ASV parametrisiert wird.

Verfahrensschritt S110 kennzeichnet, dass das Vergleichen des Atemsignalverlaufs ASV mit der zumindest einen ausgewählten Atemphase AP, AP1, AP2 des Referenzatemzyklus R ein Berechnen einer Tangente TASM des Atemsignalverlaufs ASV umfasst.

Verfahrensschritt S111 kennzeichnet, dass das Vergleichen des Atemsignalverlaufs ASV mit der zumindest einen ausgewählten Atemphase AP, AP1, AP2 des Referenzatemzyklus R ein Festlegen eines Winkelbereichs W1, W2 gemäß der zumindest einen ausgewählten Atemphase AP, AP1, AP2 des Referenzatemzyklus R umfasst und dass der Winkelbereich W1, W2 in Abhängigkeit der Änderungsrate des Referenzatemzyklus R und der Amplitude des Referenzatemzyklus R festgelegt wird.

Verfahrensschritt S112 kennzeichnet, dass ein Tangentenwinkelbereich WTASM in Abhängigkeit von dem Winkelbereich W1, W2 um eine Referenztangente des Referenzatemzyklus R festgelegt wird und dass beim Vergleichen die Tangente TASM des Atemsignalverlaufs ASV und der Tangentenwinkelbereich WTASM eingehen.

Alternativ oder zusätzlich zu Verfahrensschritt S110 kann gemäß einem weiteren nicht in Fig. 2 gezeigten Verfahrensschritt S113 das Vergleichen des Atemsignalverlaufs ASV mit der zumindest einen ausgewählten Atemphase AP, AP1, AP2 des Referenzatemzyklus R ein Berechnen eines Vektors VASM zwischen dem Atemsignalverlauf ASV und einem Mittelpunkt M des Referenzatemzyklus R umfassen, wobei ein Vektorwinkelbereich WVASM in Abhängigkeit von dem Winkelbereich W1, W2 und dem Mittelpunkt M des Referenzatemzyklus R festgelegt wird und wobei beim Vergleichen der Vektor VASM zwischen dem Atemsignalverlauf ASV und dem Mittelpunkt M des Referenzatemzyklus R sowie der Vektorwinkelbereich WVASM eingehen.

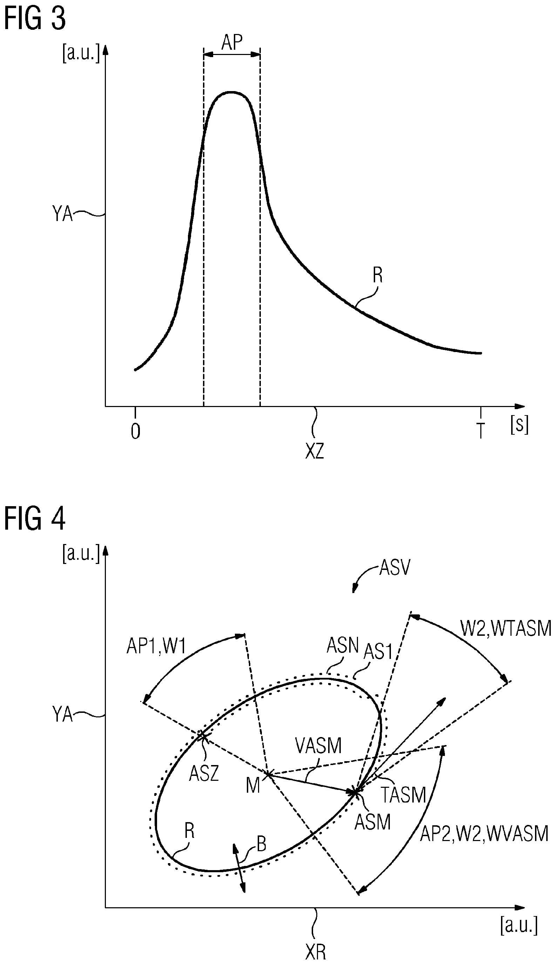

**Fig. 3** zeigt eine schematische Abbildung des Referenzatemzyklus R und der zumindest einen ausgewählten Atemphase AP in einem dritten Ausführungsbeispiel.
Der Referenzatemzyklus R kann beispielsweise derart auf einer Anzeigeeinheit der Planungseinheit 12 dem Nutzer angezeigt werden. Der Referenzatemzyklus R ist über einer y-Achse YA mit einer Amplitude des Referenzatemzyklus R und über einer x-Achse XZ mit einem Zeitverlauf des Referenzatemzyklus R abgebildet. Der Zeitpunkt T kennzeichnet eine Zyklusdauer des Referenzatemzyklus und entspricht bei einer phasenwinkelabhängigen Parametrisierung 2π. Der Nutzer kann beispielsweise mittels von Eingabemittel der Planungseinheit 12 die zumindest eine Atemphase AP auswählen.

**Fig. 4** zeigt eine schematische Abbildung des Referenzatemzyklus R und der zumindest einen ausgewählten Atemphase AP1, AP2 in einem vierten Ausführungsbeispiel. Fig. 4 illustriert schematisch und vorzugsweise zumindest teilweise den Gegenstand der Verfahrensschritte S106, S107 sowie S109 bis S113.

Der Referenzatemzyklus R kann beispielsweise derart auf einer Anzeigeeinheit der Planungseinheit 12 dem Nutzer angezeigt werden. Der Referenzatemzyklus R ist über der y-Achse YA mit der Amplitude des Referenzatemzyklus R und über einer x-Achse XR mit einer Änderungsrate des Referenzatemzyklus R abgebildet. In diesem Fall weist der Referenzatemzyklus R die Zyklusdauer auf, welche 2π beträgt. Der Referenzatemzyklus R kann daher als eine geschlossene Kreisfunktion in der durchgezogenen Linie abgebildet werden. Zusätzlich zum Referenzatemzyklus R ist der Atemsignalverlauf ASV in Fig. 4 zyklisch parametrisiert abgebildet. Der Atemsignalverlauf ASV ist in der gepunkteten Linie abgebildet.

Vorzugsweise ist eine Abweichung zwischen dem Referenzatemzyklus R und dem Atemsignalverlauf ASV gering. In diesem Ausführungsbeispiel ist der Atemsignalverlauf ASV relativ zum Referenzatemzyklus R regulär und daher ohne Schwankungen im Atemsignalverlauf ASV illustriert und die Abweichung ist gering. Wenn in einem anderen Ausführungsbeispiel eine weitere Atmung irregulär ist, schwankt typischerweise eine Abweichung zwischen einem Atemsignalverlauf der weiteren Atmung und einer weiteren Referenzatemzyklus.

Der Atemsignalverlauf ASV weist mehrere Atemsignale AS1, ASN, ASM, ASZ während der bildgebenden Messung auf, welche verschiedene Atemphasen des Referenzatemzyklus R periodisch durchlaufen können. Der Atemsignalverlauf ASV beginnt mit dem Atemsignal AS1 und endet mit dem Atemsignal ASZ. Die bildgebende Messung kann beispielsweise mit dem Atemsignal AS1 beginnen und mit dem Atemsignal ASZ enden. Eine Abtastrate der Atemerfassungseinheit 11 beeinflusst typischerweise den Abstand zwischen den jeweiligen Atemsignalen.

Fig. 4 zeigt schematisch, dass die zumindest eine ausgewählte Atemphase AP1, AP2 kürzer ist als die Zyklusdauer des Referenzatemzyklus R, weil eine Winkelabdeckung geringer ist als die Zyklusdauer, sprich geringer ist als 2π, und dass die zumindest eine Atemphase AP1, AP2 eine erste Atemphase AP1 und eine zweite Atemphase AP2 umfasst, welche unterschiedliche Atemphasen des Referenzatemzyklus R abdecken.

Beispielhaft ist in Fig. 4 schematisch gezeigt, dass das Vergleichen des Atemsignalverlaufs ASV mit der zumindest einen ausgewählten Atemphase AP1, AP2 des Referenzatemzyklus R ein Berechnen einer Tangente TASM des Atemsignalverlaufs ASV umfasst.

Ferner ist in Fig. 4 schematisch gezeigt, dass das Vergleichen des Atemsignalverlaufs ASV mit der zumindest einen ausgewählten Atemphase AP1, AP2 des Referenzatemzyklus R ein Berechnen eines Vektors VASM zwischen dem Atemsignalverlauf ASV und einem Mittelpunkt M des Referenzatemzyklus R umfasst.

Fig. 4 zeigt weiterhin schematisch, dass das Vergleichen des Atemsignalverlaufs ASV mit der zumindest einen ausgewählten Atemphase AP1, AP2 des Referenzatemzyklus R ein Festlegen eines Winkelbereichs W1, W2 gemäß der zumindest einen ausgewählten Atemphase AP1, AP2 des Referenzatemzyklus R umfasst und dass der Winkelbereich W1, W2 in Abhängigkeit der Änderungsrate des Referenzatemzyklus R und der Amplitude des Referenzatemzyklus R festgelegt wird. Der Winkelbereich W1, W2 kann insbesondere dann in Abhängigkeit der Änderungsrate des Referenzatemzyklus R und der Amplitude des Referenzatemzyklus R festgelegt werden, wenn der Referenzatemzyklus R und der Atemsignalverlauf ASV zyklisch parametrisiert sind, wodurch der Winkelbereich W1, W2 insbesondere auf derselben Basis wie der Referenzatemzyklus R und der Atemsignalverlauf ASV abgebildet werden. Im Gegensatz zu Fig. 4 ist der Referenzatemzyklus R beispielsweise in Fig. 3 nicht zyklisch parametrisiert.

Ein Tangentenwinkelbereich WTASM wird in Abhängigkeit von dem Winkelbereich W1, W2 um eine Referenztangente des Referenzatemzyklus R festgelegt. Die Referenztangente des Referenzatemzyklus R ist aus Gründen der Übersichtlichkeit nicht in Fig. 4 gezeigt und entspricht typischerweise einer Mittelinie des Tangentenwinkelbereichs WTASM. Beim Vergleichen gehen die Tangente TASM des Atemsignalverlaufs ASV und der Tangentenwinkelbereich WTASM ein.

Ein Vektorwinkelbereich WVASM wird in Abhängigkeit von dem Winkelbereich W1, W2 und dem Mittelpunkt M des Referenzatemzyklus R festgelegt. Beim Vergleichen gehen der Vektor VASM zwischen dem Atemsignalverlauf ASV und dem Mittelpunkt M des Referenzatemzyklus R sowie der Vektorwinkelbereich WVASM ein.

Grundsätzlich kann die Referenztangente, der Tangentenwinkelbereich WTASM und/oder die Tangente TASM sowie der Vektor VASM und der Vektorwinkelbereich WVASM zueinander registriert und/oder auf denselben Startpunkt für das Vergleichen verschoben werden.

Die Tangente TASM und der Vektor VASM werden in diesem Ausführungsbeispiel relativ zum Atemsignal ASM abgebildet. Das binäre Vergleichsergebnis beträgt in Abhängigkeit des Atemsignals ASM Wahr und die Datenerfassung wird ausgelöst. Im Gegensatz dazu beträgt das binäre Vergleichsergebnis im Falle des Atemsignals ASN Falsch und es werden keine Projektionsdaten erfasst.

Der Vektor VASM ermöglicht insbesondere ein Ermitteln einer zeitlichen Abweichung. Die Tangente TASM ermöglicht insbesondere ein Ermitteln einer Abweichung in der Regularität. Grundsätzlich ist es denkbar, dass mittels des Vektors VASM und/oder der Tangente TASM die zeitliche Abweichung und/oder die Abweichung in der Regularität ermittelt werden.

Das Generieren des binären Vergleichsergebnisses umfasst insbesondere ein Überprüfen, ob der Atemsignalverlauf ASV relativ zu der zumindest einen ausgewählten Atemphase AP1, AP2 die Kriterien erfüllt oder nicht. Die Kriterien umfassen insbesondere einen Toleranzbereich für eine Abweichung zwischen dem Atemsignalverlauf ASV und der zumindest einen ausgewählten Atemphase AP1, AP2.

Der Toleranzbereich kann grundsätzlich dem Winkelbereich W1, W2 entsprechen. In diesem Fall wird die Abweichung zwischen dem Atemsignalverlauf ASV und der zumindest einen ausgewählten Atemphase AP1, AP2 beispielsweise zwischen dem in Fig. 4 beispielhaft gewählten Atemsignal ASM und einer Mittelinie der zumindest einen ausgewählten Atemphase AP1, AP2 ermittelt. Beim Vergleichen kann grundsätzlich ermittelt werden, ob das Atemsignal ASM in den Winkelbereich W1, W2 der zumindest einen ausgewählten Atemphase AP1, AP2 fällt oder nicht. Alternativ oder zusätzlich ist es denkbar, dass mittels eines Anfangs und/oder eines Endes der zumindest einen ausgewählten Atemphase AP1, AP2 überprüft wird, ob der Atemsignalverlauf ASV relativ zu dem zumindest einen ausgewählten Atemphase AP1, AP2 die Kriterien erfüllt oder nicht. Wenn der Atemsignalverlauf ASV relativ zu der zumindest einen ausgewählten Atemphase AP1, AP2 die Kriterien erfüllt, weist typischerweise das binäre Vergleichsergebnis Wahr auf und die Datenerfassung wird ausgelöst. Gemäß dem Atemsignalverlauf ASV werden in der ersten Atemphase AP1 zweimal und in der zweiten Atemphase AP2 einmal Projektionsdaten erfasst.
Der Toleranzbereich kann weiterhin ein radial um den Referenzatemzyklus R gezogenes Band B mit einer insbesondere in Abhängigkeit von der Regularität der Atmung des Patienten P gewählten Dicke aufweisen, in welchem der Atemsignalverlauf ASV sich typischerweise befindet, wenn das binäre Vergleichsergebnis Wahr aufweist. Aus Gründen der Übersichtlichkeit ist das Band B nur an einer Stelle des Referenzatemzyklus R angedeutet.

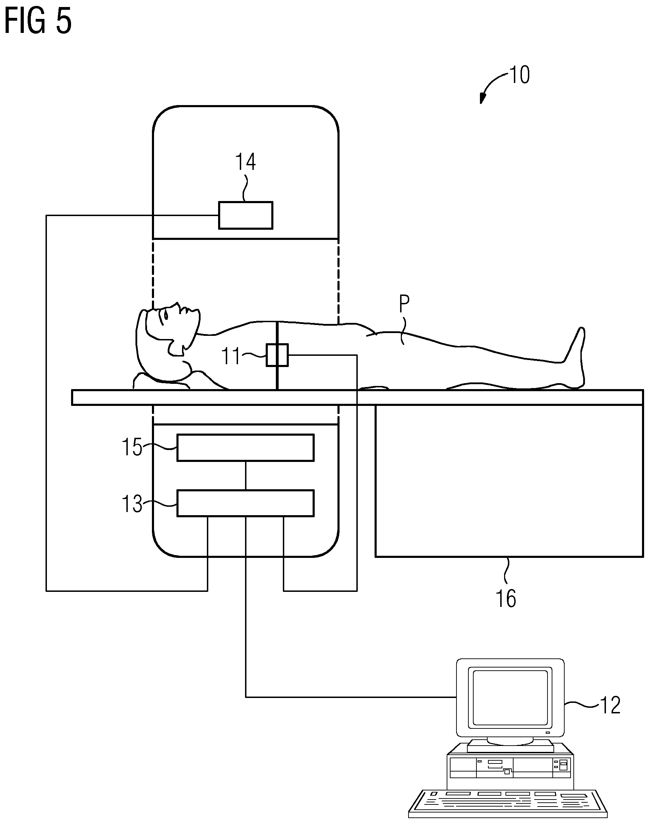

**Fig. 5** zeigt ein Computertomographiesystem 10 schematisch in einem Querschnitt. Das Computertomographiesystem 10 weist die Atemerfassungseinheit 11, die Planungseinheit 12 und die Steuereinheit 13 auf. Zusätzlich weist das Computertomographiesystem 10 eine Röntgenröhre 14 und einen Röntgendetektor 15 auf. Für die Datenerfassung ist die Röntgenröhre 14 zu einem Emittieren von Röntgenstrahlung ausgebildet, welche nach einem Durchdringen eines Patienten P von dem Röntgendetektor 15 als Projektionsdaten erfasst werden. Der Patient P ist auf einer Patientenliege 16 gelagert.

In diesem Ausführungsbeispiel ist die Atemerfassungseinheit 11 als Atemgurt ausgebildet. Die Planungseinheit 12 weist eine Anzeigeeinheit und Eingabemittel auf. Die Steuereinheit 13 ist mit der Atemerfassungseinheit 11, der Planungseinheit 12, der Röntgenröhre 14 und dem Röntgendetektor 15 verbunden, insbesondere für das Erfassen des Atemzyklus gemäß Verfahrensschritt S100, für das Bereitstellen des Atemzyklus gemäß Verfahrensschritt S101, für das Auswählen der zumindest einen Atemphase S102, für das Übertragen der zumindest einen ausgewählten Atemphase gemäß Verfahrensschritt S103 und für das Durchführen der bildgebenden Messung gemäß Verfahrensschritt S104, S104A, S104B, S104C. Eine Recheneinheit kann als die Steuereinheit 13 ausgebildet sein.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren für ein Durchführen einer bildgebenden Messung eines Patienten (P) in einem Computertomographiesystem (10) mit folgenden Schritten:
- Erfassen eines Atemzyklus des Patienten (P) mittels einer Atemerfassungseinheit (11) des Computertomographiesystems (10),
- Bereitstellen des Atemzyklus des Patienten (P) in einer Planungseinheit (12) des Computertomographiesystems (10) als Referenzatemzyklus (R),
- Auswählen zumindest einer Atemphase (AP, AP1, AP2) des Referenzatemzyklus (R) mittels der Planungseinheit (12), wobei die zumindest eine ausgewählte Atemphase (AP, AP1, AP2) kürzer ist als eine Zyklusdauer des Referenzatemzyklus (R),
- Übertragen der zumindest einen ausgewählten Atemphase (AP, AP1, AP2) gemeinsam mit dem Referenzatemzyklus (R) in eine Steuereinheit (13) des Computertomographiesystems (10),
- Durchführen der bildgebenden Messung in dem Computertomographiesystem (10),
-- wobei eine Atmung des Patienten (P) mittels der Atemerfassungseinheit (11) erfasst und als Atemsignalverlauf (ASV) in die Steuereinheit (13) übertragen wird,
-- wobei der Atemsignalverlauf (ASV) mit der zumindest einen ausgewählten Atemphase (AP, AP1, AP2) des Referenzatemzyklus (R) in der Steuereinheit (13) verglichen wird, wodurch ein binäres Vergleichsergebnis generiert wird,
-- wobei in Abhängigkeit von dem binären Vergleichsergebnis eine Datenerfassung in dem Computertomographiesystem (10) ausgelöst wird, wodurch Projektionsdaten in der zumindest einen Atemphase (AP, AP1, AP2) erfasst werden.

2. Verfahren nach Anspruch 1, wobei nach dem Durchführen der bildgebenden Messung ein medizinisches Bild in der zumindest einen Atemphase (AP, AP1, AP2) unter Verwendung der Projektionsdaten rekonstruiert und bereitgestellt wird.

3. Verfahren nach einem der vorhergehenden Ansprüche, wobei die zumindest eine Atemphase (AP, AP1, AP2) eine erste Atemphase (AP1) und eine zweite Atemphase (AP2) umfasst, welche unterschiedliche Atemphasen des Referenzatemzyklus (R) abdecken, und wobei die Datenerfassung gemäß der ersten Atemphase (AP1) und gemäß der zweiten Atemphase (AP2) ausgelöst wird.

4. Verfahren nach einem der vorhergehenden Ansprüche, wobei die bildgebende Messung in einem Messbereich mit einer ersten z-Position und einer zweiten z-Position durchgeführt wird und wobei die Projektionsdaten gemäß der zumindest einen Atemphase (AP, AP1, AP2) an der ersten z-Position und an der zweiten z-Position erfasst werden.

5. Verfahren nach den Ansprüchen 3 bis 4.

6. Verfahren nach einem der vorhergehenden Ansprüche, wobei für das Vergleichen der Referenzatemzyklus (R) zyklisch in Abhängigkeit einer Änderungsrate des Referenzatemzyklus (R) und einer Amplitude des Referenzatemzyklus (R) parametrisiert wird und wobei der Atemsignalverlauf (ASV) zyklisch in Abhängigkeit einer Änderungsrate des Atemsignalverlaufs (ASV) und einer Amplitude des Atemsignalverlaufs (ASV) parametrisiert wird.

7. Verfahren nach einem der vorhergehenden Ansprüche, wobei das Vergleichen des Atemsignalverlaufs (ASV) mit der zumindest einen ausgewählten Atemphase (AP, AP1, AP2) des Referenzatemzyklus (R) ein Berechnen einer Tangente (TASM) des Atemsignalverlaufs (ASV) umfasst.

8. Verfahren nach einem der vorhergehenden Ansprüche, wobei das Vergleichen des Atemsignalverlaufs (ASV) mit der zumindest einen ausgewählten Atemphase (AP, AP1, AP2) des Referenzatemzyklus (R) ein Berechnen eines Vektors (VASM) zwischen dem Atemsignalverlauf (ASV) und einem Mittelpunkt (M) des Referenzatemzyklus (R) umfasst.

9. Verfahren nach einem der vorhergehenden Ansprüche, wobei das Vergleichen des Atemsignalverlaufs (ASV) mit der zumindest einen ausgewählten Atemphase (AP, AP1, AP2) des Referenzatemzyklus (R) ein Festlegen eines Winkelbereichs (W1, W2) gemäß der zumindest einen ausgewählten Atemphase (AP, AP1, AP2) des Referenzatemzyklus (R) umfasst und wobei der Winkelbereich (W1, W2) in Abhängigkeit der Änderungsrate des Referenzatemzyklus (R) und der Amplitude des Referenzatemzyklus (R) festgelegt wird.

10. Verfahren nach den Ansprüchen 7 und 9, wobei ein Tangentenwinkelbereich (WTASM) in Abhängigkeit von dem Winkelbereich (W1, W2) um eine Referenztangente des Referenzatemzyklus (R) festgelegt wird und wobei beim Vergleichen die Tangente (TASM) des Atemsignalverlaufs (ASV) und der Tangentenwinkelbereich (WTASM) eingehen.

11. Verfahren nach den Ansprüchen 8 und 9, wobei ein Vektorwinkelbereich (WVASM) in Abhängigkeit von dem Winkelbereich (W1, W2) und dem Mittelpunkt (M) des Referenzatemzyklus (R) festgelegt wird und wobei beim Vergleichen der Vektor (VASM) zwischen dem Atemsignalverlauf (ASV) und dem Mittelpunkt (M) des Referenzatemzyklus (R) sowie der Vektorwinkelbereich (WVASM) eingehen.

12. Verfahren nach den Ansprüchen 10 und 11.

13. Computertomographiesystem (10) aufweisend
- die Atemerfassungseinheit (11),
- die Planungseinheit (12) und
- die Steuereinheit (13), wobei das Computertomographiesystem (10) nach einem der vorhergehenden Ansprüche ausgebildet ist.

14. Computerprogrammprodukt, welches direkt in einen Speicher einer Recheneinheit ladbar ist, mit Programmcodemitteln, um ein Verfahren nach einem der Ansprüche 1 bis 12 auszuführen, wenn das Computerprogrammprodukt in der Recheneinheit ausgeführt wird.
